(19) Europäisches Patentamt
European Patent Office
Office européen des brevets

(11) Publication number: **0 086 822 B1**

(12) # EUROPEAN PATENT SPECIFICATION

(45) Date of publication of patent specification: **04.03.87**

(51) Int. Cl.⁴: **C 07 C 29/00**, C 07 C 41/01, C 07 C 17/10

(21) Application number: **82902800.0**

(22) Date of filing: **12.08.82**

(86) International application number: **PCT/US82/01099**

(87) International publication number: **WO 83/00859 17.03.83 Gazette 83/07**

(54) **PROCESS FOR THE PRODUCTION OF METHYL HALIDES AND METHYL ALCOHOL FROM METHANE.**

(30) Priority: **01.09.81 US 298390**

(43) Date of publication of application: **31.08.83 Bulletin 83/35**

(45) Publication of the grant of the patent: **04.03.87 Bulletin 87/10**

(84) Designated Contracting States: **DE FR GB NL**

(56) References cited:
**GB-A-1 017 152**
**GB-A-1 104 294**
**SU-A- 388 529**
**US-A-1 086 381**
**US-A-1 591 984**
**US-A-1 688 726**
**US-A-1 723 442**
**US-A-2 156 039**
**US-A-3 172 915**
**US-A-3 562 321**

(73) Proprietor: **Olah, George Andrew**
**2252 Gloaming Way**
**Beverly Hills California (US)**

(72) Inventor: **Olah, George Andrew**
**2252 Gloaming Way**
**Beverly Hills California (US)**

(74) Representative: **McCallum, William Potter et al**
**19 Royal Exchange Square**
**Glasgow G1 3AE (GB)**

(56) References cited:

**OLAH, FRIEDEL-CRAFTS AND RELATED REACTIONS, INTERSCIENCE PUBLISHERS, NEW YORK, 1963, page 26**
**NAFION PAMPHLET, DUPONT, April 1978 "PERFLUOROSULFONIC ACID PRODUCTS"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 086 822 B1

**Description**

Technical field

This invention relates to solid acidic or metal catalyst-promoted halogenation of methane to produce methyl monohalides in high selectivity. Concurrent or simultaneous hydrolysis provides methyl alcohol and/or dimethyl ether in good yields.

Background art

The selective conversion of methane into its monofunctional derivatives, such as methyl halides or methyl alcohol, is highly desirable, but in practice has not been achieved on any practical basis.

The chloronation of methane is an industrial process practiced on a large scale. The reaction is a strongly exothermic one which takes place via free radicals and is generally conducted without supplying heat and usually in the absence of a catalyst at 400—450°C under slightly elevated pressures. The chlorination is normally thermally initiated via homolysis of chlorine molecules to chlorine atoms; the process can also be operated photochemically. For surveys of these processes, it is appropriate to refer to F. Asinger "Paraffins. Chemistry and Technology", Pergamon Press, New York, 1968; M. L. Poutsma "Methods in Free Radical Chemistry", Vol. II, E. S. Huyser, Ed., M. Dekker, New York, 1969; and R. Weissermel and H. J. Arpe "Industrial Organic Chemistry", Verlag Chemie, 1978, pp. 46—47. By these reactions, all of the possible chlorinated methanes are usually formed together when an equimolar $Cl_2/CH_4$ ratio is employed:

$$CH_4+Cl_2 \xrightarrow[-HCl]{440°C} \underset{37\%}{CH_3Cl}+\underset{41\%}{CH_2Cl_2}+\underset{19\%}{CHCl_3}+\underset{3\%}{CCl_4}$$

If methyl chloride is the preferred product, a large excess of methane (approx. tenfold) must be used, as methyl chloride is more rapidly chlorinated than methane under free radical conditions. There are normally many by-products of the chlorination of methane, such as hexachloroethane and small amounts of trichloroethylene.

Methyl alcohol is increasingly important not only as a chemical raw material and building block for such products as formaldehyde, acetic acid, vinyl acetate, ethylene glycol and others, but also via its condensation reactions to give gasoline or hydrocarbons, such as olefins, aromatics and the like. Its direct use as a transportation fuel is also gaining importance. A whole scope of so-called $C_1$ chemistry is based primarily on methyl alcohol.

Methyl alcohol, once produced from wood fermentation (i.e., wood alcohol), is, at the present time, nearly exclusively produced from CO and $H_2$ (synthesis gas) derived from coal or natural gas. Coal or methane first must be converted in an energy consuming step into syn-gas, which is then, in a second energy consuming step under pressure and generally forcing conditions, converted into methyl alcohol. Clearly, direct oxidative conversion of methane into methyl alcohol would be highly desirable. Despite continued efforts no such process was, however, previously achieved on a practical scale.

The oxidation of methane generally is not selective. In the past, many attempted oxidations concentrated on manufacturing formaldehyde from methane. The low rate of reaction of $CH_4$ at temperatures below 600°C coupled with the high rate of decomposition of formaldehyde above 600°C is probably the reason that no industrial process has been developed to date. Decomposition of formaldehyde could only be avoided by extremely short residence times. Such a process has been recently described involving partial oxidation of methane to methyl alcohol and formaldehyde. The residence time is $1.55\times10^{-3}$ sec. and the pressure 60 atm., respectively (Huels). However, oxidation of methane, similarly to chlorination, is free radical chain reaction, which explains the observed lack of selectivity.

I have previously described in the *Journal of the American Chemical Society*, Vol. 95, 7686 (1973) that, under specific conditions, alkanes can undergo electrophilic chlorination and chlorinolysis. With a $SbF_5$ catalyst in $SO_2ClF$ solution at −78° or at room temperature with a reaction time of 24 hours, methane was transformed qualitatively to methyl chloride. No practical yields were obtained. $AlCl_3$ catalyst gave under similar conditions 1% methyl chloride. These reactions clearly did not represent a practical method for the chlorination of methane. GB—A—1 017 152 discloses a process for the production of flourinated methanes "and especially chlorofluorinated methanes". In fact, the primary object of the disclosed invention is the production of highly flourinated and/or chlorofluorinated compounds directly from methane i.e. the production of poly-halogenated methanes with substantially complete replacement of hydrogen atoms linked to carbon. In addition, the process described in the patent requires the reaction of methane with hydrogen fluoride and chlorine in the presence of one or more halogenated hydrocarbons at a temperature between 200 and 700°C, preferably between 400 and 500°C. All of the examples recited in the patent were carried out at temperatures at or above 400°C. There is no disclosure of the production of methyl monohalides from methane and halogen at the relatively low temperature of 100 to 325°C, let alone using Group IVB, VB or VIB transition metal oxides or oxyhalides as supported catalysts in the highly selective production of methyl monohalides.

US—A—2 156 039 discloses a process of chlorinating methane and its homologues by reacting the hydrocarbon with chlorine at high temperatures over a permeable contact-plane yielding a mixture of mono and multichlorinated end products. Metals, such as nickel in fine mesh form as well as thin porous plates of glass, asbestos etc, are suggested as suitable catalysts for the reaction. Although the patent states that the starting materials are preheated to temperatures between 220—400°C, the actual chlorination reaction of the methane takes place at significantly higher temperatures of the order of 450—480°C. Moreover the process of this prior patent produces 40% or more poly chlorinated hydrocarbon. Thus the process disclosed provides little or no selectivity and certainly no significant selectivity in favour of monohalogenation; there is no disclosure of halogenation let alone selective monohalogenation at relatively low temperatures in the range from 100 to 325°C; and there is no suggestion at all of the use of any Group IVB, VB or VIB metal oxides or oxyhalides supported on a chalconite carrier.

US—A—1 591 984 discloses a process for the chlorination of hydrocarbons by reacting hydrocarbons with chlorine in the presence of oxygen at high temperatures. The use of a catalyst is optional. Suggested catalysts include metal halides such as iron chloride, cupric chloride, and chlorides of the alkaline earth metals. The temperature range disclosed is between 300 and 650°C, the preferred range being between 400 and 550°C. Again there is no disclosure of the use of an oxide or oxyhalide of a Group IVB, VB or VIB metal catalyst supported on a chalconite carrier. Moreover the prior process requires the presence of oxygen and in general temperatures well above 350°C. Lastly, there is no suggestion at all that the prior process could in any way be selective, let alone that it could be selective for methyl monohalides as is the present invention.

US—A—1 723 442 discloses the chlorination of methane by reacting methane with chlorine in the presence of steam as a diluent at a high temperature range of 400—500°C. As with US—A—1 591 984, the use of a catalyst is optional with suggested catalysts including ferric chloride, cupric chloride, and halides of the alkaline earths. The optional catalyst of the prior process is a non-supported metal halide, and there is no disclosure or suggestion of a catalyst comprising an oxide or oxyhalide of a Group IVB, VB or IVB metal supported on a chalconite carrier. Again there is no clear indication as to the identity and proportions of the reaction products, it being merely mentioned that one possible constituent of the reaction products could be methyl chloride. Moreover this prior patent suggests that the (only) way of modifying the nature of the reaction products obtained is by regulating the proportion of chlorine and methane in accordance with the products of chlorination which are to be obtained. There is no suggestion at all that a high degree of selectivity in respect of one particular reacton product could be achieved, let alone that this could be achieved by the use of particular catalysts.

GB—A—1 104 294 discloses inter alia a process for brominating alkanes optionally in the presence of a catalyst. As already mentioned hereinbefore conventional halogenation of alkanes is a free radical type of process. In this prior patent the only catalysts mentioned are tetraethyl lead or other catalysts generating free radicals in the reaction.

US—A—3 172 915 discloses a process of chlorinating methane using a ferric chloride catalyst. In this case even where the main reaction product is monochloromethane this is obtained only at a level of 52% with as much as 35% of dichloromethane and 13% of trichloromethane as shown in the example. Thus again there is not the slightest suggestion of the possibility of obtaining high selectivity let alone that this can be achieved with particular catalysts. Neither does the closely related US—A—3 356 321 of the same inventors suggest this.

It is an object of the present invention to avoid or minimize one or more of the above disadvantages.

The present invention provides a process for the catalytic production of methyl monohalides from methane which process comprises reacting methane with a halogen selected from chlorine, bromine or iodine at an elevated temperature in the presence of a catalyst characterized in that said catalyst is a solid acid catalyst selected from the Group IVB, VB or VIB transition metal oxides or oxyhalides and is supported on a chalconite carrier, and the reaction is carried out at a temperature of from 100 to 325°C so as to produce methyl monohalides with from 85 to 99% selectivity.

In a further aspect the present invention provides a process for producing methyl alcohol from methane, halogen selected from chlorine, bromine or iodine, and water at an elevated temperature using a catalyst characterized in that the catalyst is selected from the Group IVB, VB or VIB transition metal oxides or oxyhalides and is supported on a chalconite carrier and the reaction is carried out on a mixture of methane, halogen selected from chlorine, bromine or iodine, and water at a temperature of from 100 to 320°C.

In my co-pending Application No. 85200869.7 divided out herefrom, there is provided a process for the selective catalytic production of methyl monohalides from methane carried out at temperatures between about 100 to 300°C in the presence of a halogen using as the catalyst a supported Group VIII metal catalyst.

The catalysts

Either (i) solid strongly acidic catalysts or (ii) supported Group VIII metal (particularly platinum and palladium) catalysts are capable of catalyzing the gas-phase halogenation of methane predominantly to methyl monohalides in 85 to 99% selectivity. Subsequent or concurrent catalytic hydrolysis gives methyl alcohol and/or dimethyl ether.

A particularly useful class of solid, strongly acidic catalysts are those derived from halides, oxyhalides,

oxides, sulfides and oxysulfides of metals, particularly transition metals of Groups IV, V, VI, VIII of the Periodic Table, such as of tantalum, niobium, zirconium, tungsten, titanium, chromium, and the like, or mixtures thereof, deposited on suitable chalconite carriers, such as alumina, zirconia or silica-alumina. These catalysts are capable of effecting the ready conversion of methane to methyl halides.

As noted in Olah, G. A. "Friedal-Crafts Chemistry," N.Y., Wiley-Interscience, 1973, p. 343—344, the elements of Group VIA such as oxygen, sulfur, selenium or tellurium, have been called "chalcogens", and compounds containing these elements are called "chalconites", "chalcogenides" or "chalcides". A variety of solid oxides and sulfides, especially those comprising alumina, silica and mixtures of alumina and silica, either natural or synthetic, in which other oxides such as chromia, magnesia, molybdena, thoria, tungstic oxide, zirconia, etc., may also be present, as well as sulfides of molybdenum are useful chalcide carriers. Many naturally occurring compositions exist for use as the carriers including: bauxite, floridin, Georgia clay, and other natural aluminosilicates.

Synthetic chalconites other than those of the silica-alumina type, representative of the chalconite carriers are: BeO, $Cr_2O_3$, $P_2O_5$, $ThO_2$, $TiO_2$, $Al_2(SO_4)_3$ (which may be regarded as $Al_2O_3 \cdot 3SO_3$), $Al_2O_3 \cdot Cr_2O_3$, $Al_2O_3$, $Fe_2O_3$, $Al_2O_3 \cdot CoO$, $Al_2O_3 \cdot MnO$, $Al_2O_3 \cdot V_2O_3$, $Al_2O_3 \cdot Mo_2O_3$, $Cr_2O_3 \cdot Fe_2O_3$, $MoS_2$, and $MoS_3$.

The acidic chalconite supports are physically and chemically stable. They are generally catalytically active at only higher temperatures, as their acidity is not great enough to lead them to form stable complexes with unsaturated compounds, as do the aluminum halides, for example.

The supported Group VIII metal catalysts include the various Group VIII metals supported on suitable chalconite carriers. Particularly useful platinum and palladium supported on alumina, silica, barium sulfate or related carriers.

The process conditions

It is my invention that a practical process has been found for the selective acidic or metal catalyzed halogenation of methane to methyl monohalides (chloride, bromide, iodide or fluoride).

The acidic halogenations are carried out over solid acidic catalysts, particularly supported metal-based catalysts, preferably selected from transition metal halides, oxides or oxyhalides, such as those of iron, tantalum, niobium or zirconium, on alumina, baria, or other neutral oxides, barium sulfate or related carriers, at temperatures between about 100 to 500°C, preferably between 200 and 325°C.

Metal catalyzed reactions are carried out over supported metal catalysts, preferably of the Group VIII metals, on alumina, baria, or other neutral oxides, barium sulfate or related carriers, at temperatures between 100 and 350°C, preferably between 200—300°C.

The solid acidic reactions are considered to take place via surface catalytic activation of chlorine, bromine, or iodine to an electrophilic halogen species. The selective nature of the reactions is reflected by the fact that even when using an excess of halogen (methane to chlorine ratio of 1:2 to as high as 1:8), methyl halides are formed in 85 to >99% selectivity over methylene halides.

At the same time, highly selective (99%) monochlorination is readily achieved when reacting excess methane (methane to chlorine ratio from 1:1 to 8:1) over the same catalysts. Excess methane under these conditions is also a diluent for the system and eliminates any potentially explosive mixtures.

If is a further part of my invention that similar selective monohalogenation of methane was also discovered to be possible using supported metal catalysts on alumina, baria, or other neutral oxides, barium sulfate or related carriers.

In a similar fashion bromination and iodination of methane can also be achieved both in acid catalyzed electrophilic and metal promoted halogenations.

Fluorination of methane is also possible, but necessitates high dilution, preferably with an inert gas, and handling difficulties associated with the use of elemental fluorine.

Hydrogen chloride, bromide and iodide by-products of the reactions can be readily recycled via oxyhalogenation and thus reused.

Methyl halides formed in the selective halogenations disclosed in my invention can be used according to my co-pending application serial No. 290,292, now United States Patent No. 4,373,109 in the production of lower olefins and hydrocarbons of the gasoline range, when treated over bifunctional acidic-basic catalysts.

The methyl halides readily obtainable in high yield and selectivity according to my invention are also conveniently used via their hydrolysis either under thermal or catalytic conditions, to produce methyl alcohol and/or dimethyl ether. The conversion of methane to methyl alcohol can be accordingly carried out as a two-step process, but also can be practiced as a single-step process converting methane directly to methyl alcohol (and/or dimethyl ether) when reacting methane in the presence of water (steam) with halogens. In this application, acidic oxides or oxyhalides compatible with water, are preferred.

$$CH_4 \xrightarrow[H_2O]{X2} CH_3OH + 2HX$$

As hydrogen halides (X=Cl, Br, I) can be readily recycled via oxyhalogenation, the disclosed process

represents a viable and energy saving alternative to the production of methyl alcohol from CO and $H_2$, i.e., syn-gas.

In all of the reactions discussed, the catalyst is preferably present in an amount of 0.1 to 25% based on the amount of methane.

The following examples are illustrative and are set forth for the purpose of illustration only and are not to be construed as limiting the scope of the invention in any manner. In the related Tables, the product compositions have been normalized, even if not stated, to provide 100 percent conversion, excluding unreacted methane which can be recycled.

Example 1

A mixture of methane and chlorine in the molar ratios indicated, was reacted over a 20% tantalum oxyfluoride catalyst deposited on alumina in a continuous flow reactor at 235°C to 300°C, respectively. The conversions per pass and product compositions were as follows:

| | | | | | | |
|---|---|---|---|---|---|---|
| Reaction temp. °C | 235 | 235 | 300 | 240 | 240 | 240 |
| $CH_4$:$Cl_2$ ratio | 1:2 | 1:4 | 1:2 | 2:1 | 4:1 | 8:1 |
| % Conversion | 13* | 88* | 40* | 91** | 95** | 99** |

*based on methane
**based on chlorine

| | % Product composition | | | | | |
|---|---|---|---|---|---|---|
| methyl chloride | 75 | 96.5 | 90 | 99 | 99 | 99 |
| methylene chloride | — | trace | 10 | trace | trace | trace |
| ethane | 25 | — | trace | — | — | — |
| ethyl chloride | — | 3.5 | — | — | — | — |

Example 2

Methane and chlorine in a molar ratio of 1:2 were passed at 250°C over a 20% niobium oxyfluoride catalyst supported on alumina. The product composition obtained with 39% conversion was the following:

| % Product composition | |
|---|---|
| methyl chloride | 98 |
| methylene chloride | 2 |

Example 3

Methane and chlorine in a molar ratio of 1:4 were passed over a 20% zirconium oxyfluoride catalyst on alumina in a continuous flow reactor at 270°C. With 30% conversion per pass the following product composition was obtained:

| % Product composition | |
|---|---|
| methyl chloride | 96.5 |
| methylene chloride | 3.5 |

Example 4

A mixture of methane and chlorine in a milar ratio of 1:4 was reacted over a 20% antimony oxyfluoride catalyst deposited on alumina at 250°C. A 19% conversion per pass gave the following product composition:

| % Product composition | |
|---|---|
| methyl chloride | 86 |
| methylene chloride | 5 |
| ethyl chloride | 9 |

Example 5

A 1:2 molar mixture of methane and bromine was reacted in the presence of a 20% antimony oxyfluoride catalyst supported on alumina at 200°C. With a 36% conversion per pass, the following product composition was obtained:

| | % Product composition |
|---|---|
| methyl bromide | 87 |
| ethylene bromide | 3 |
| ethyl bromide | 0.5 |
| butanes | 9.5 |

Example 6

A 1:3 molar mixture of methane and chlorine was passed at 250°C over a 1% iron catalyst supported on alumina with a 59% conversion per pass. The following product composition was obtained:

| | % Product composition |
|---|---|
| methyl chloride | 97 |
| ethyl chloride | 3 |

Example 7

Methane and chlorine were passed over a 0.5% platinum catalyst deposited on alumina. The conversions and product composition obtained were the following:

| | | | | |
|---|---|---|---|---|
| Reaction temp. °C | 200 | 200 | 250 | 250 |
| $CH_4$:$Cl_2$ ratio | 1:2 | 1:1 | 2:1 | 4:1 |
| % Conversion | 50* | 44* | 74** | 82** |

* based on methane
**based on chlorine

| | % Product composition | | | |
|---|---|---|---|---|
| methyl chloride | 92 | 99 | 99 | 99 |
| methylene chloride | 8 | trace | trace | trace |

Example 8

Methane and chlorine were passed at 200°C over a 0.5 palladium catalyst deposited on barium sulfate. The conversions and product composition obtained were the following:

| | | |
|---|---|---|
| Reaction temp. °C | 200 | 200 |
| $CH_4$:$Cl_2$ ratio | 1:2* | 2:1** |
| % Conversion | 29 | 69 |

* based on methane
** based on chlorine

| | % Product composition | |
|---|---|---|
| methyl chloride | 99 | 99 |
| ethylene chloride | trace | trace |

Example 9

A 1:1:1 molar mixture of methane, bromine and water was reacted over a 20% tantalum oxyfluoride catalyst supported on alumina at 260°C. With a 77% conversion, the following product composition was obtained:

| | |
|---|---|
| methyl bromide | 64% |
| methyl alcohol + dimethyl ether | 31% |
| ethyl bromide | 3% |
| $C_3$+$C_4$ | 2% |

Example 10

A 1:1:1 molar mixture of methane, chlorine and water was reacted over a 20% tungsten oxide on alumina catalyst at 250°C. With a 49% conversion, the following product distribution was obtained.

| | |
|---|---|
| methyl chloride | 68% |
| methyl alcohol + dimethyl ether | 24% |
| ethyl chloride | 6% |
| methylene chloride | trace |
| $C_3$+$C_4$ | 2% |

Example 11

A 1:5 molar mixture of methyl chloride and steam was passed, in a continuous flow reactor, over a catalyst composed of 10% zinc oxide on alumina containing 10% aluminum hydroxide at 385°C. A 29% conversion per pass of methyl alcohol was obtained with 98% selectivity.

Example 12

Under conditions of Example 11, when carrying out the reaction with a catalyst composed of 10% magnesium oxide on alumina containing 10% aluminum hydroxide at 420°C, a 21% conversion to methyl alcohol was obtained with 98% selectivity.

Example 13

Under conditions of Example 11, when carrying out the reaction with a catalyst composed of 10% titanium oxide on alumina containing 10% aluminum hydroxide, a 18% conversion to methyl alcohol was obtained with 98% selectivity.

**Claims**

1. A process for the catalytic production of methyl monohalides from methane which process comprises reacting methane with a halogen selected from chlorine, bromine or iodine at an elevated temperature in the presence of a catalyst characterized in that said catalyst is a solid acid catalyst selected from the Group IVB, VB or VIB transition metal oxides or oxyhalides and is supported on a chalconite carrier, and the reaction is carried out at a temperature of from 100 to 325°C so as to produce methyl monohalides with from 85 to 99% selectivity.

2. A process according to Claim 1 wherein the transition metal is tantalum, niobium, zirconium or titanium and the chalconite carrier is alumina.

3. A process for producing methyl alcohol from methane, halogen selected from chlorine, bromine or iodine, and water at an elevated temperature using a catalyst characterized in that the catalyst is selected from the Group IVB, VB or VIB transition metal oxides or oxyhalides and is supported on a chalconite carrier and the reaction is carried out on a mixture of methane, halogen selected from chlorine, bromine or iodine, and water at a temperature of from 100 to 320°C.

4. A process according to any one of Claims 1 to 3 wherein the catalyst is present in an amount of from 0.1 to 25% by weight based on the amount of methane.

**Patentansprüche**

1. Verfahren zur katalytischen Herstellung von Methylmonohaliden aus Methan, wobei man Methan mit einem Halogen aus der Gruppe Chlor, Brom oder Jod bei einer erhöhten Temperatur in Anwesenheit eines Katalysators umsetzt, dadurch gekennzeichnet, daß der Katalysator ein fester Säurekatalysator aus den Oxiden oder Oxyhalogeniden der Übergangsmetalle der Gruppe IVB, VB oder VIB ist und auf einem Chalkonid-Träger gestützt ist, und daß die Reaktion bei einer Temperatur von 100 bis 325°C durchgeführt wird, um Methylmonohalide mit 85 bis 99%iger Selektivität zu erzeugen.

2. Verfahren nach Anspruch 1, wobei das Ubergangsmetall Tantal, Niob, Zirkon oder Titan und der Chalkonid-Träger Aluminiumoxid ist.

3. Verfahren zur Herstellung von Methylalkohol aus Metan, Halogen aus der Gruppe Chlor, Brom oder Jod, und Wasser bei erhöhter Temperatur unter Verwendung eines Katalysators, dadurch gekennzeichnet, daß der Katalysator aus den Oxiden oder Oxyhaliden der Übergangsmetalle der Gruppen IVB, VB oder VIB ausgewählt ist und auf einem Chalkonid-Träger gestützt ist und die Reaktion an einer Mischung aus Methan, Halogen aus der Gruppe Chlor, Brom oder Jod, und Wasser bei einer Temperatur von 100 bis 320°C durchgeführt wird.

4. Verfahren nach einem der Ansprüche 1 bis 3, wobei der Katalysator in einer Menge von 0,1 bis 25 Gewichts-%, bezogen auf die Menge an Methan, vorliegt.

**Revendications**

1. Un procédé pour la production catalytique de monohalogénures de méthyle à partir de méthane, lequel procédé comprend la réaction du méthane avec un halogène choisi parmi le chlore, le brome ou l'iode à une température élevée en présence d'un catalyseur, caractérisé en ce que ledit catalyseur est un catalyseur acide solide choisi parmi les oxydes ou oxyhalogénures des métaux de transition du groupe IVB, VB ou VIB et est fixé sur un support de chalconite et la réaction est effectuée à une température de 100 à 325°C pour produire des monohalogénures de méthyle avec une sélectivité de 85 à 99%.

2. Un procédé selon la revendication 1 dans lequel le métal de transition est le tantale, le niobium, le zirconium ou le titane et le support de chalconite est l'alumine.

3. Un procédé pour la production d'alcool méthylique à partir de méthane, d'un halogène choisi parmi le chlore, le brome ou l'iode et d'eau à une température élevée utilisant un catalyseur, caractérisé en ce que le catalyseur est choisi parmi les oxydes ou oxyhalogénures de métaux de transition des groupes IVB, VB ou VIB et est fixé sur un support de chalconite et la réaction est effectuée sur un mélange de méthane, d'un halogène choisi parmi le chlore, le brome ou l'iode et d'eau à une température de 100 à 320°C.

4. Un procédé selon l'une quelconque des revendications 1 à 3 dans lequel le catalyseur est présent en une quantité de 0,1 à 25% en poids par rapport à la quantité de méthane.